(19) Europäisches Patentamt
European Patent Office
Office européen des brevets

(11) Veröffentlichungsnummer: **0 212 210**
**A2**

(12) # EUROPÄISCHE PATENTANMELDUNG

(21) Anmeldenummer: **86109411.8**

(51) Int. Cl.4: **C07D 303/16** , C07D 301/14

(22) Anmeldetag: **10.07.86**

(30) Priorität: **05.08.85 DE 3528004**

(43) Veröffentlichungstag der Anmeldung:
**04.03.87 Patentblatt 87/10**

(84) Benannte Vertragsstaaten:
**AT CH DE FR GB IT LI NL SE**

(71) Anmelder: **Degussa Aktiengesellschaft**
**Weissfrauenstrasse 9**
**D-6000 Frankfurt am Main 1(DE)**

(72) Erfinder: **Boehme, Georg**
**Nordring 49**
**D-6458 Rodenbach 1(DE)**
Erfinder: **Hofen, Willi**
**Südring 54**
**D-6458 Rodenbach 1(DE)**
Erfinder: **Grund, Andreas, Dr.**
**Rilkeweg 15**
**D-6100 Darmstadt(DE)**
Erfinder: **Petsch, Heinrich**
**Tulpenstrasse 23**
**D-6450 Hanau 8(DE)**
Erfinder: **Prescher, Günter, Dr.**
**Liesingstrasse 2**
**D-6450 Hanau 9(DE)**

(54) **Verfahren zur Herstellung von cycloaliphatischen Diepoxiden.**

(57) Cycloaliphatische Diepoxide der Formel

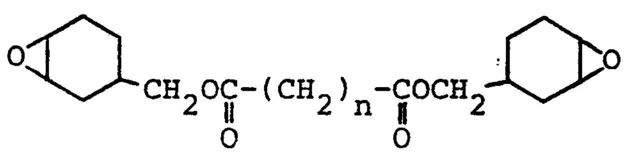

werden in technisch einfacher Form durch Epoxydieren der entsprechenden Diolefine mit Perpropionsäure in benzolischer Lösung, die auch in ungereinigter Form mit bestimmtem Maximalgehalten an Wasserstoffperoxid, Wasser und Mineralsäure verwendet werden kann, hergestellt.

EP 0 212 210 A2

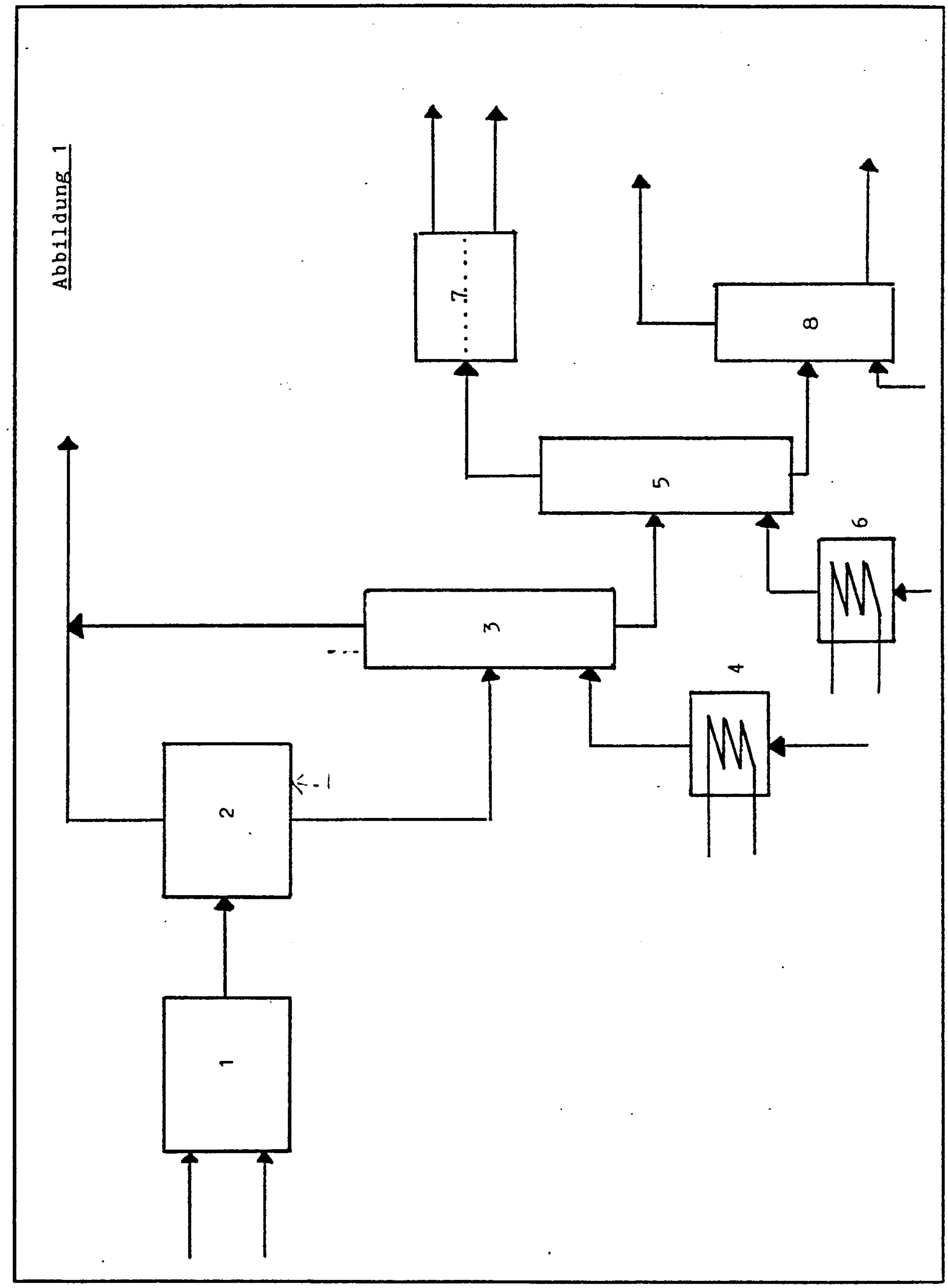
Abbildung 1
1
2
3
4
5
6
7
8

## Verfahren zur Herstellung von cycloaliphatischen Diepoxiden

Die vorliegende Erfindung betrifft ein Verfahren zur Herstellung von cycloaliphatischen Diepoxiden der folgenden Formel

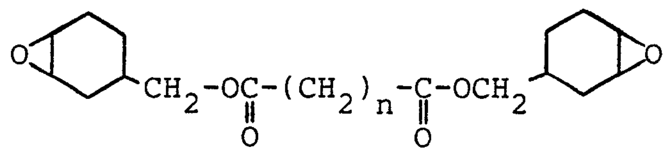

durch Epoxidation von Diolefinen der Formel

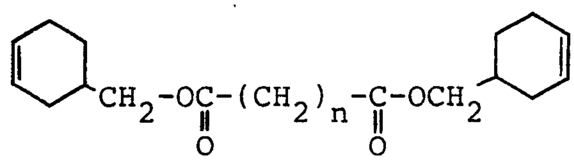

in der n eine Zahl von 0 bis 30 bedeutet, mit einer Percarbonsäure in organischem Lösungsmittel sowie zur Aufarbeitung des dabei entstehenden Reaktionsgemisches.

Cycloaliphatische Diepoxide obenstehender Struktur finden zunehmend Verwendung als Bestandteil von Epoxidharzen, die sich vor allem als Isolàtions-und Vergußmaterial auf dem Elektro-und Elektroniksektor sowie für strahlungshärt bare Lack-und Beschichtungssysteme eignen. Gerade letztere sind besonders umweltfreundlich, da sie frei von Lösungsmitteln sind.

Es ist seit langem bekannt, Epoxide durch Umsetzung von Olefinen mit Chlor im alkalischen Medium und nachfolgender Behandlung mit Basen herzustellen (Ullmann's Enzyklopädie der technischen Chemie, 3. Auflage, Band 10, Seite 565). Der wesentliche Nachteil deises Verfahrens sind die erheblichen Mengen umweltbelastender Abwässer, die bei diesem Prozeß zwangsweise anfallen.

Es ist ferner bekannt, daß Ethylen mit guten Ausbeuten in der Gasphase mit molekularem Sauerstoff an einem silberhaltigen Katalysator epoxidiert werden kann. Wegen seiner mangelnden Selektivität ist jedoch dieses Verfahren für andere Olefine ungeeignet.

Weiterhin lassen sich Olefine durch Umsetzung mit Hydroperoxiden, die aus Kohlenwasserstoffen, wie z.B. Isobutan oder Ethylbenzol, durch Oxidation mit Luft erhältlich sind (US-PS 3 351 635), in Gegenwart eines Katalysators, der Vanadin-, Molybdän-oder Wolframverbindungen enthält, in entsprechende Epoxide überführen. Diese Methode hat den gewichtigen Nachteil, daß neben der erforderlichen Abtrennung des Katalysatorsystems der aus dem Hydroperoxid als Koppelprodukt in äquimolarem Maßstab anfallende Alkohol, sofern er nicht wirtschaftlich verwertbar ist, nur unter erheblichem technischen Aufwand in das Hydroperoxid rückgeführt werden kann.

Durch Anwendung der "Prileschajew-Reaktion" (N. Prileschajew, Ber. dtsch. chem. Ges. 42, 4811 - (1909)) können die zuvor erwähnten Nachteile teilweise vermieden bzw. vermindert werden.

Diese Reaktion beinhaltet im wesentlichen die Umsetzung eines Olefins mit einer organischen Percarbonsäure. Allerdings führt auch hierbei z.B. die Verwendung von Perameisensäure, die darüberhinaus in höheren Konzentrationen detonationsfähige Gemische ausbildet, zu erheblichen Abwassermengen, die einer gewissenhaften Entsorgung bedürfen. Auch ein Einsatz von Peressigsäure in wäßrigem Medium führt zu großen Mengen verdünnter Essigsäure, die auf wirtschaftliche Weise nicht aufkonzentriert und rückgeführt werden können. Falls die Peressigsäure, wie häufig notwendig wegen der Produktstabilität, während des Prozesses mit Alkalicarbonatlösung abgepuffert und/oder nach der Reaktion mit Alkalihydroxidlösung neutralisiert wird, fallen stark salzhaltige, die Umwelt belastende Abwässer an.

Diepoxide ließen sich im allgemeinen durch Umsetzung der entsprechenden Olefine mit Acetaldehydmonoperacetat oder mit Peressigsäure in Äthylacetat oder Aceton nach dem Verfahren der US-PS 2 750 395 herstellen. Dieses zunächst attraktiv erscheinende Verfahren hatte den großen Nachteil, daß als Koppelprodukt Essigsäure anfiel, die mit erheblichem Aufwand aus dem Reaktionsgemisch abgetrennt und gereinigt werden mußte. Außerdem lagen die Ausbeuten an Diepoxiden bei nur 69 bis 88 % und die Epoxidgehalte bei nur 70 bis 87 % in den erhaltenen Produkten. Darüberhinaus ist der Prozeß der Acetaldehydoxidation nicht ungefährlich, da explosive Zwischenprodukte auftreten können.

Im allgemeinen vertritt die Fachwelt über Epoxidation mit Persäuren die Meinung, daß die derartig mit Persäuren erhaltenen Reaktionsgemische aufgrund ihres Gehalts an Wasser und Säuren, z.B. Essigsäure, sehr leicht mit den gebildeten Epoxiden unter Bildung von Nebenprodukten, wie Glykolen, Glykolmono-und -diestern reagieren, siehe DE-AS 15 43 032.

Epoxidationsverfahren, die z.B. Perameisen-bis Perpropionsäure verwendeten, galten daher als besonders schwierig durchführbar in saurem Milieu, da hierdurch Aufspaltung des Oxiranringes eintrat, siehe DE-PS 29 16 834.

In diesem Zusammenhang ist es auch zu verstehen, daß in der DE-OS 31 01 037 und EP-OS 056 932, die die Herstellung von n-Alkyloziranen mittels Perpropionsäure beschreiben, im Anspruch 1 eine Percarbonsäurelösung mit einem Mineralsäuregehalt kleiner als 50 ppm beansprucht wird. Laut Beschreibung im Text soll der Mineralsäuregehalt bevorzugt sogar kleiner als 10 ppm betragen. Diese Verfahren bezogen sich auf die Herstellung von Monoepoxiden.

Nach dem genannten Stand der Technik war aber zu erwarten, daß sich Diepoxide noch schlechter mit Percarbonsäure herstellen lassen würden, da diese wegen der zwei vorhandenen Epoxid-Teilstrukturen besonders leicht zu Folgereaktionen neigen.

Aufgabe der Erfindung ist die Herstelllung von Diepoxiden cycloaliphatischer Dicarbonsäureester mit Hilfe von Perpropionsäure in guten Ausbeuten und hoher Reinheit.

Es wurde nun gefunden, daß sich diese Aufgabe lösen läßt durch Umsetzen von Diolefinen der Formel

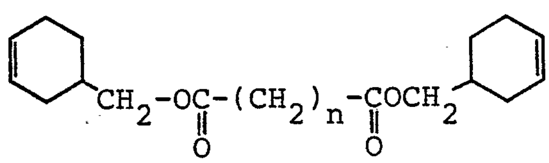

in der n eine Zahl zwischen 0 bis 30 bedeutet, mit Percarbonsäuren in organischer Lösung, wenn man die genannten Diolefine mit einer Lösung von Perpropionsäure in Benzol im Molverhältnis von 1 : 2 bis 1 : 3 (Diolefin zu Perpropionsäure) bei einer Temperatur von 10 bis 100 °C umsetzt.

Sehr geeignet sind Diolefine, in denen n eine Zahl zwischen 0 und 11, besonders bevorzugt n = 4, bedeutet.

Perpropionsäure kann z.B. gemäß dem in DE-PS 25 19 289 beschriebenen Verfahren hergestellt werden, indem man wäßriges Wasserstoffperoxid mit Propionsäure in Gegenwart von Schwefelsäure umsetzt und anschließend die entstandene Perpropionsäure mit Benzol aus dem Reaktionsgemisch extrahiert. Die auf diese Weise erhaltene Perpropionsäure in benzolischer Lösung kann noch weiter gereinigt werden, um den Restgehalt an Schwefelsäure, Wasser und Wasserstoffperoxid zu verringern, siehe z.B. DE-PS 25 19 290. Bevorzugt ist aber eine Perpropionsäurelösung, die keiner weiteren Reinigung bedarf; mit anderen Worten, der Rohextrakt aus der Perpropionsäureherstellung kann als solcher direkt eingesetzt werden. Dies führt zu einem erheblich verringerten technischen Aufwand.

Es kann daher eine Perpropionsäurelösung in Benzol verwendet werden, die bis 1,5 Gewichtsprozent Wasserstoffperoxid, 1,5 Gewichtsprozent Wasser und bis zu 800 ppm Mineralsäure enthält.

Nach dem erfindungsgemäßen Verfahren wird das Diolefin vorzugsweise als solches, oder aber auch verdünnt in einem geeigneten Lösungsmittel, z.B. Benzol, eingesetzt, wobei man die Konzentration in einem weiten Bereich frei wählt.

Die Lösungen der Perpropionsäure, die u.a. noch aus Propionsäure bestehen, können zwischen 10 und 30 Gewichtsprozent der Persäure enthalten. Vorzugsweise werden Lösungen mit einem Persäuregehalt von ca. 20 Gewichtsprozent eingesetzt. Ein bevorzugtes Molverhältnis von Diolefin zu Perpropionsäure liegt bei 1 : 2 bis 1 : 2,4. Besonders bevorzugt ist auch ein Persäureüberschuß von 3 bis 15, bevorzugt 3 bis 10, Molprozent.

Die Umsetzung findet bevorzugt bei Temperaturen von 20 bis 50°C statt. Das erfindungsgemäße Verfahren läßt sich unter verschiedenen Drücken durchführen; im allgemeinen wird unter Normaldruck gearbeitet, das Verfahren läßt sich aber auch bei Über-oder Unterdruck durchführen.

Die Umsetzung kann sowohl diskontinuierlich oder kontinuierlich in für diese Art der Reaktion geeigneten Reaktoren, wie Rührkesseln, Rührkesselkaskaden, Rühr-oder Schlaufenreaktoren erfolgen, wobei die Reaktionswärme auf beliebige Weise, z.B. Siedekühlung oder innen-bzw. außenliegende Kühleinrichtungen, abgeführt wird.

Geeignete Werkstoffe für die Reaktionsapparate zur Durchführung des erfindungsgemäßen Verfahrens sind z.B. Glas, Edelstahl oder emailliertes Material.

Die Perpropionsäure wird mit dem Diolefin auf beliebige Art zusammengebracht. So kann man beide Reaktionsteilnehmer zusammen oder nacheinander in beliebiger Reihenfolge in den Reaktor einbringen. Bei diskontinuierlicher Arbeitsweise wird vorzugsweise das Diolefin vorgelegt und die Persäure unter Kontrolle der Reaktionstemperatur zudosiert; es kann aber ebensogut umgekehrt verfahren werden, d.h. man legt die Persäure vor und dosiert das Olefin temperaturkontrolliert zu. Erfolgt die Umsetzung kontinuierlich, so können beide Reaktanten getrennt oder gemeinsam dem Reaktor zugeführt werden. Bei Verwendung mehrerer hintereinandergeschalteter Reaktoren, wie z.B. einer Rührkesselkaskade oder einer Folge von Rührkesseln mit einem Rohrreaktor als Nachreaktor, kann man sowohl Persäure-als auch Diolefinzugabe auf mehrere Reaktoren verteilen.

Die als Ausgangsmaterialien verwendeten Diolefine werden nach bekannten Methoden aus entsprechenden Dicarbonsäure durch Veresterung mit 3-Cyclohexenylmethanol gewonnen, siehe US-PS 2,863,881.

Als Dicarbonsäure können eingesetzt werden Oxalsäure, Malonsäure, Bernsteinsäure, Glutarsäure, Adipinsäure, Pimelinsäure, Sebacinsäure bis hin zu Dodecan-und Tridecandisäure. Besonders bevorzugt ist der Diester der Adipinsäure. Als cycloaliphatische ungesättigte Alkoholkomponente wird 3-Cyclohexenylmethanol verwendet, welches durch Umsetzung von Acrolein mit Butadien zu 3-Cyclohexen-1-carboxaldehyd und dessen Reduktion zum Alkohol nach dem Fachmann bekannten Methoden leicht erhältlich ist.

Bei dem beschriebenen Prozeß kann das Diolefin vorzugsweise als solches, aber auch verdünnt in einem geeigneten Lösungsmittel eingesetzt werden, wobei die Konzentration in einem weiten Bereich frei gewählt werden kann. Als Lösungsmittel kommt bevorzugt Benzol, aber auch Toluol, Chlorbenzol bzw. halogenierte Kohlenwasserstoffe, wie Methylenchlorid, Chloroform, Tetrachlorkohlenstoff, in Frage.

Die Lösungen der Perpropionsäure, die überwiegend aus Benzol, Propionsäure und Perpropionsäure bestehen, können zwischen 10 und 30 Gewichtsprozent der Persäure enthalten. Vorzugsweise werden Lösungen mit einem Persäuregehalt von ca. 20 Gewichtsprozent eingesetzt.

Nach dem erfindungsgemäßen Verfahren ist eine kontinuierliche Arbeitsweise besonders vorteilhaft. Gemäß dieser wird also das cycloaliphatische Diolefin bzw. seine Lösung mit einer Lösung von Perpropionsäure in Benzol im Molverhältnis 1 : 2 bis 1 : 3 bei den genannten Temperaturen von 10 bis 100 °C (bevorzugt 20 bis 50 °C) in ein Reaktionssystem eingespeist, das aus einer Folge von 1 bis 4 ideal durchmischten Reaktoren und einem Nachreaktor besteht, wobei man die Verweilzeit so einstellt, daß der Umsatz, bezogen auf eingesetzte olefinische Doppelbindungen nach dem oder den ideal durchmischten Reaktor(en) mindestens 80 Molprozent und nach dem Nachreaktor minestens 95, bevorzugt größer als 98 Molprozent, beträgt. Anschließend wird das den Nachreaktor verlassende Reaktionsgemisch in einer Kombination von Desillations-und Desorptionsschritten von Benzol, Propionsäure, unumgesetzter Perpropionsäure sowie sonstigen flüchtigen Bestandteilen befreit. Diese Auftrennung des Reaktionsgemisches kann, da das gebildete Diepoxid die Komponente mit dem höchsten Siedepunkt des Gemisches darstellt, nach einer der folgenden Varianten durchgeführt werden.

Variante 1 (diskontinuierlich)

Gemäß dieser werden die einzelnen Bestandteile des Reaktionsgemisches in der Reihenfolge ihrer Siedepunkt einzeln oder als Gemische destillativ oder destillativ und desorptiv entfernt. Hierbei gehen Fraktionen von Benzol, Resten Perpropionsäure, Propionsäure und sonstige leicht flüchtige Bestandteile über. Als Sumpf verbleibt das Diepoxid. Das ab getrennte Benzol sowie die Propionsäure können gegebenenfalls nach weiteren Reinigungsschritten in die Persäureherstellung rückgeführt werden.

Variante 2 (kontonuierlich, Abbildung 1)

Nach dieser kontinuierlich durchzuführenden Variante werden zunächst, nachdem das Reaktionsgemisch die Reaktionseinheit 1 verlassen hat, Benzol, Propionsäure und nichumgesetzte Perpropionsäure größtenteils in der ein-oder mehrstufigen Destillationseinheit 2 entfernt. Diese besteht aus geeigneten Apparaten, wie Dünnschicht-, Fallfilm-oder Umlaufverdampfern. Es ist vorteilhaft, unter vermindertem Druck von 0,5 bis 600, vorzugsweise 10 bis 300 mbar, zu destillieren - (Temperatur des Heizmediums 50 bis 150 °C). Die mittleren Verweilzeiten, bezogen auf die einzelnen Stufen der Verdampfung, liegen bei maximal 10 Minuten, bevorzugt werden Verweilzeiten von maximal 5 Minuten. Anschließend wird nach dem erfindungsgemäßen Verfahren die im Rohprodukt verbliebene Menge Propionsäure in der Desorptioneinheit 3 mit Benzoldampf, der im Verdampfer 4 generiert wird, desorptiv entfernt. Die Brüden aus der Desorptionseinheit 3 können entweder an der Destillationseinheit 2 vorbeigeführt oder durch sie hindurchgeführt werden. Nach diesem Schritt werden aus dem Diepoxid die verbliebenen Spuren Benzol mit Wasserdampf aus dem Verdampfer 6 in der Desorptionseinheit 5 und/oder Stickstoff bzw. sonstigen Inertgasen in der Desorptionseinheit 8 desorbiert. Es ist besonders bevorzugt, zunächst mit Wasserdampf und anschließend mit Inertgasen zu desorbieren. Das aus der Desorptionseinheit 5 stammende Kondensat trennt sich im Phasentrenner 7 in eine organische Phase und Wasser, welches dem Verdampfer 6, gegebenenfalls nach Ergänzung, zurückgeführt werden kann. Die organische Phase, die überwiegend Benzol und Propionsäure enthält, wird gegebenenfalls nach weiterer Aufarbeitung der Perpropionsäureherstellung oder Epoxidation zugeführt. Ebenfalls werden die aus den Destillations-bzw. Desorptionseinheiten 2 und 3 stammenden Kondensatströme, die im wesentlichen aus Benzol, nichtumgesetzter Perpropion-und Propionsäure bestehen, nach weiterer Auftrennung -siehe Abbildung 3 -, deren Beschreibung später erfolgt, in die Persäureherstellung bzw. Epoxidation rückgeführt.

Variante 3 (kontinuierlich, Abbildung 2)

Gemäß der kontinuierlich anzuwendenden Variante 3 werden, wie bei Variante 2, Benzol, unumgesetzte Perpropionsäure und Propionsäure in der ein-oder mehrstufigen Destillationseinheit 2 entfernt. Anschließend wird in der Desorptionseinheit 3 restliche Propionsäure mit Benzoldampf desorbiert. Zur Entfernung von verbliebenen Spuren an Propionsäure wird nun das Rohepoxid mit wäßrigen Alkalien in der Extraktion 9 und anschließend mit Wasser in der ein-oder mehrstufigen Extraktionseinheit 10 gewaschen. Geeignete Apparate für diese Schritte sind Extraktionskolonnen verschiedener Bauart oder auch Mixer-Settler-Einheiten, deren Betriebsweise und Auslegung dem Fachmann wohlbekannt sind. Als wäßrige Alkalilösungen können Lösungen, wie z.B. NaOH, KOH, $Na_2CO_3$, $K_2CO_3$, $NaHCO_3$, $KHCO_3$, $NH_3$ usw. eingesetzt werden, wobei deren Konzentration in einem weiten Bereich frei wählbar ist. Bevorzugt wird eine NaOH-Lösung mit einer Konzentration zwischen 0,01 und 10 Gewichtsprozent, besonders zwischen 0,1 und 2,0 Gewichtsprozent. Bei Einsatz von Mixer-Settler-Einheiten für die Wäsche mit Wasser kann das Wasser im Gegenstrom geführt werden, es kann aber auch jede Einheit mit Frischwasser betrieben werden. Vorteilhaft wird ein Teil des Abwassers aus den Mixer-Settler-Einheiten zum Ansetzen der Alkali-Lösung verwendet. Die Alkali-und Wasserwäsche kann in einem Temperaturbereich von 10 bis 90 °C betrieben werden, bevorzugt werden Temperaturen von 30 bis 70 °C. In der Alkali-Wäsche beträgt das Gewichtsverhältnis zwischen durchgesetztem Epoxid zu Alkalilösung 1 : 1 bis 1 : 100, in der Wasserwäsche liegt das Verhältnis von Epoxiddurchsatz zu Wasserdurchsatz bei 1 : 1 bis 1 : 100.

Im Anschluß an die Wasserwäsche erfolgt die weitere Aufarbeitung durch Desorption mit Wasserdampf und/oder Inertgas wie bei Variante 2 beschrieben.

Die bei allen Varianten durch Kombination von Destillations-und Desorptionsschritten anfallenden Kondensate, die überwiegend aus Benzol, unumgesetzter Perpropionsäure. Propionsäure und sonstigen Leichtsiedern bestehen, werden nach dem erfindungsgemäßen Verfahren in eine aus einer oder mehreren Kolonnen bestehende Destillationseinheit 11 (Abbildung 3) überführt. Diese liefert als Kopfprodukt Benzol und gegebenenfalls weitere Leichtsieder. Ersteres wird gegebenenfalls nach weiterer Reinigung in 12 in das Herstellungsverfahren der Perpropionsäure zurückgeführt. Im Sumpf der Destillationseinheit 11 fällt ein Gemisch aus Propionsäure, Perpropionsäure und Benzol mit einem Benzolanteil von 5 bis 35 Gewichtsprozent, bezogen auf die Sumpfmischung, an. Dieses Gemisch wird einer weiteren Destillationseinheit 13 zugeführt, in der man die Gesamtmenge des zugeführten Benzols und der Perpropionsäure mit Anteilen Propionsäure über Kopf abzieht und dabei eine Konzentration von Perpropionsäure im Destillat von 25 Gewichtsprozent nicht überschreitet und dieses Kopfprodukt in das Herstellungsverfahren der Perpropionsäure oder in die Umsetzung des Diolefins mit Perpropionsäure zurückführt. Als Sumpfprodukt in Kolonne 13 fällt Propionsäure an,

die nach weiterer Aufarbeitung wie Reindestillation in die Herstellung der Perpropionsäure, gegebenenfalls nach Ergänzung, zurückgeführt wird. Besonders vorteilhaft ist es, die in 13 anfallende Propionsäure dampfförmig oberhalb des Sumpfes abzuziehen und zu kondensieren, da hierdurch der weitere Reinigungsschritt entfällt.

Erfindungsgemäß werden alle destillativen Aufarbeitungsschritte vorzugsweise unter vermindertem Druck, z.B. 0,5 bis 600 mbar, durchgeführt. Kolonnen, in denen Benzol oder Propionsäure als Kopfprodukt anfällt, können ebenso bei Normaldruck betrieben werden.

Das erfindungsgemäße Verfahren bietet eine Reihe von überraschenden Vorteilen.

Mit Hilfe der sogenannten Prileschajew-Reaktion ist es nach diesem Verfahren möglich, die genannten Diepoxide im technischen Maßstab auf gefahrlose Weise in hoher Ausbeute herzustellen. Das auf diese Weise erhaltene Produkt zeichnet sich durch außerordentliche Reinheit, hohen Epoxidgehalt, Geruchlosigkeit und helle Farbe aus. Ebenfalls besonders niedrig ist der Gehalt an Monoepoxid sowie ionischen Verunreinigungen, wodurch ein Produkt mit deutlich besseren Eigenschaften als nach anderen Verfahren hergestellten Diepoxiden vorstehender Struktur zur Verfügung steht. Gerade für Anwendungen im Bereich der Mikroelektronik werden an die Reinheit der dort einzusetzenden Diepoxide Qualitätsanforderungen gestellt, wie sie nach dem erfindungsgemäßen Verfahren erfüllt werden.

Das beschriebene Verfahren ist wirtschaftlich, da alle Hilfsmedien rückgeführt werden. Das Verfahren ist besonders umweltfreundlich, da aus dem Oxidationsmittel lediglich Wasser als Abfall entsteht; darüber hinaus fallen nur geringe Mengen an sonstigen Abwässern, Leichtsiedern und Destillationsrückständen an, die unproblematisch und gefahrlos entsorgt werden können.

Erfindungsgemäß sind nur kurze Reaktionszeiten nötig, was die technische Durchführung besonders wirtschaftlich gestaltet.

Es war überraschend und nicht vorhersehbar, daß die Umsetzung der vorgenannten Diolefine mit einer rohen Perpropionsäure, die noch Mineralsäure, Wasser und Wasserstoffperoxid in den vorgenannten Konzentrationen enthält, bei weitestgehender Unterdrückung von Neben-und Folgereaktionen durchführbar ist. Weiterhin war nich vorhersehbar, daß die dabei anfallenden Reaktionsgemische erfindungsgemäß destillativ oder destillativ und desorptiv aufarbeitbar sind, ohne daß sich dadurch der Epoxidgehalt des Produkts merklich verringert.

In Tabelle 1 wird der Unterschied wesentlicher Produkteigenschaften des Bis-(epoxycyclo-hexenylmethyl)-adipates, das einmal nach dem bekannten Verfahren mit Peressigsäure in Ethylacetat und dann nach dem erfindungsgemäßen Verfahren gewonnen wurde, herausgestellt; es ergibt sich nach dem erfindungsgemäßen Verfahren eine Erhöhung des Epoxidgehaltes bei gleichzeitiger erheblicher Verringerung der Farbzahl und der Viskosität. Das mit Peressigsäure hergestellte Produkt ist eine handelsübliche Ware.

Tabelle 1:

| | Peressigsäure/ Ethylacetat | Perpropionsäure |
|---|---|---|
| Epoxidgehalt (Val/kg) | 4,81 | 5,11 - 5,19 |
| Farbzahl (Hazen) | 100 - 150 | 30 |
| Viskosität (mPas.s) | 653 | 495 |

Die Prozentangaben in den Beispielen 1 bis 11 sind Gewichtsprozente.

Beispiel 1 (diskontinuierlich)

Unter Rühren und Kühlung tropfte man zu 100 g (0,36 Mol) Oxalsäure-bis-(cyclohexenylmethyl)-ester 335 g (0,79 Mol) einer 21,3 %igen Lösung von Perpropionsäure in Benzol innerhalb 60 Minuten zu, wobei die Temperatur auf 20 °C gehalten

wurde. Die Persäure war nach dem Verfahren der DE-PS 25 19 289 hergestellt worden und enthielt 0,54 Gew.-% $H_2O_2$; 0,86 Gew.-% $H_2O$ und 580 ppm $H_2SO_4$. Sie wurde für die Beispiele 1 -9 verwandt. Nach der Zugabe ließ man 180 Minuten bei 40 °C nachreagieren.

Die erhaltene klare Lösung wurde bei 95 °C und 120 mbar über einen Dünnschichtverdampfer gegeben, wobei gleichzeitig im Gegenstrom Benzoldampf geführt wurde. Im Sumpf fiel das Rohepoxid an. Dieses wurde erneut bei 95 °C und 65 mbar über einen Dünnschichtverdampfer gegeben; gleichzeitig wurde im Gegenstrom mit Wasserdampf desorbiert.

Als Sumpfabzug erhielt man 102 g Oxalsäure-bis-(epoxycyclohexylmethyl)-ester mit folgenden Kennzahlen:

Viskosität (mPas.s): 209

Epoxidgehalt (val/kg): 6,42

Farbzahl (Hazen): 70

Beispiele 2 bis 9 (diskontinuierlich)

Wie bei Beispiel 1 wurden die nachfolgenden Ester von aliphatischen Dicarbonsäuren und Tetrahydrobenzylalkohol mit Perpropionsäure in Benzol epoxidiert und aufgearbeitet: Malonsäure-bis-(cyclohexenylmethyl)-ester, Bernsteinsäure-bis-(cyclohexenylmethyl)-ester, Glutarsäure-bis-(cyclohexenylmethyl)-ester, Adipinsäure-bis-(cyclohexenylmethyl)-ester, Pimelinsäure-bis-(cyclohexenylmethyl)-ester, Decan-di-säure-bis-(cyclohexenylmethyl)-ester, Dodecan-di-säure-bis-(cyclohexenylmethyl)-ester und Tri-decan-di-säure-bis-(cyclohexenylmethyl)-ester.

Die Versuchsergebnisse sind der Tabelle 2 zu entnehmen:

Tabelle 2:

| Beispiel | Diolefin 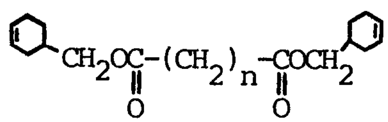 | Diepoxid 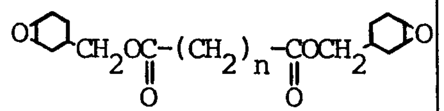 | Ausbeute (%) | Epoxid-Gehalt (val/kg) | Viskosität (mPas) | Farbe (Hazen) |
|---|---|---|---|---|---|---|
| 2 | n = 1 | n = 1 | 97 | 5,82 | 1038 | 70 |
| 3 | n = 2 | n = 2 | 92 | 5,61 | 1371 | 70 |
| 4 | n = 3 | n = 3 | 90 | 5,23 | 611 | 150 |
| 5 | n = 4 | n = 4 | 94 | 5,20 | 540 | 100 |
| 6 | n = 5 | n = 5 | 91 | 5,00 | 381 | 100 |
| 7 | n = 8 | n = 8 | 90 | 4,71 | 311 | 100 |
| 8 | n = 10 | n = 10 | 96 | 4,59 | 350 | 40 |
| 9 | n = 11 | n = 11 | 83 | 4,31 | 340 | 50 |

Beispiel 10 (kontinuierlich)

In den ersten Rührkessel einer Reaktionseinheit, bestehend aus zwei Rührkesseln vom Volumen jeweils 900 ml sowie einem als Rohrreaktor ausgebildetem Nachreaktor, der bei einer Länge von 3 m ein Volumen von 1900 ml hat, wurden stündlich 3,34 Mol Perpropionsäure in Benzol (ca. 22 %ig) und 1,46 Mol Adipinsäure-bis-(cyclohexenylmethyl)-ester eingespeist, was einem Molverhältnis (Persäure zu Diolefin) 2,29 : 1 entspricht. Die Perpropionsäure, die auch hier nach dem Verfahren der DE-PS 25 19 289 hergestellt wurde, enthielt 0,56 Gewichtsprozent $H_2O_2$; 0,90 Gewichtsprozent $H_2O$ und 620 ppm $H_2SO_4$. Sie wurde bei den Beispielen 10 und 11 eingesetzt.

Die Reaktionstemperatur betrug im ersten Reaktor 40 °C, im zweiten Reaktor 40 °C und Nachreaktor 36 bis 41 °C. Die Umsätze an Olefin betrugen nach der Rührkesselkaskade 98 %, nach dem Rohrreaktor 99,8 %. Gemäß Aufarbeitungsvariante 3 wurden zunächst in einem Sambay-Verdampfer mit der Fläche 0,065 m² bei einer Temperature von 90 °C und einem Druck von 100 mbar Benzol, Perpropionsäure und Propionsäure abgetrennt. Restliche Propionsäure wurde in einem zweiten Verdampfer gleichen Typs und gleicher Fläche bei 90 °C und 100 mbar bei einem Durchsatz von 315 g/h Benzoldampf desorbiert. Die Brüden des zweiten Verdampfers wurden vollständig im Gegenstrom zum Produktstrom in den ersten Verdampfer geführt.

Das so als Sumpf erhaltene Rohepoxid wurde nun in einem Mixer-Settler-System mit 1 % iger Natronlauge (180 ml/h) und anschließend in einer Folge von drei Mixer-Settler-Einheiten mit Wasser (jeweils 180 ml/h) gewaschen.

Nachfolgend wurde das Epoxid in zwei Desorptionseinheiten, bestehend aus jeweils einem Sambay-Verdampfer (Fläche 0,065 m²) mit einer am Sumpfabzug angesetzten Füllkörperkolonne (NW 25, Länge 90 cm, Raschigringe) bei 100 mbar mit 49 g/h Wasserdampf und bei 20 mbar mit 36 g/h Stickstoff bei Temperaturen von 90 °C behandelt.

Als Produkt fielen stündlich 523 g Diepoxid mit folgenden Kennzahlen an:

Viskosität (mPa.s): 510

Epoxid-Gehalt (val/kg): 5,22

Farbzahl (Hazen): 60.

Beispiel 11 (kontinuierlich)

In den ersten Rührkessel einer Reaktionseinheit, bestehend aus zwei Rührkesseln vom Volumen von jeweils 1500 ml sowie einem als Rohrreaktor ausgebildeten Nachreaktor, der bei einer Länge von 3 m ein Volumen von 1900 ml hat, wurden stündlich 3,31 Mol Perpropionsäure in Benzol (ca. 22 %ig) und 1,47 Mol Adipinsäure-bis-(cyclohexenylmethyl)-ester eingespeist, was einem Molverhältnis (Persäure zu Diolefin) 2,26 : 1 entspricht.

Die Reaktionstemperatur betrug im ersten Reaktor 40 °C, im zweiten Reaktor 40 °C und im Nachreaktor 36 bis 40 °C. Die Umsätze an Olefin betrugen nach der Rührkesselkaskade 98,5 %, nach dem Rohrreaktor 99,9 %. Gemäß Aufarbeitungsvariante 3 wurden zunächst in einem Sambay-Verdampfer mit der Fläche von 0,065 m² bei einer Temperatur von 90 °C und einem Druck von 100 mbar Benzol, Perpropionsäure und Propionsäure abgetrennt. Restliche Propionsäure wurde in einem zweiten Verdampfer gleichen Typs und gleicher Fläche bei 90 °C und 100 mbar bei einem Durchsatz von 315 g/h Benzoldampf desorbiert. Die Brüden des zweiten verdampfers wurden nicht durch den ersten Verdampfer geführt.

Das so erhaltene Rohepoxid wurde nun in einem Mixer-Settler-System mit 1 %iger Natronlauge - (180 ml/h) und anschlie ßend in einer Folge von drei Mixer-Settler-Einheiten mit Wasser (jeweils 180 ml/h) gewaschen.

Nachfolgend wurde das Epoxid in zwei Desorptionseinheiten, bestehend aus jeweils einem Sambay-Verdampfer (Fläche 0,065 m²), mit einer am Sumpfabzug angesetzten Füllkörperkolonne (NW 25, Länge 90 cm , Raschigringe) bei 100 mbar mit 49 g/h Wasserdampf und bei 20 mbar mit 36 g/h Stickstoff bei Temperaturen von 90 °C behandelt.

Als Produkt fielen stündlich 524 g Diepoxid mit folgenden Kennzahlen an:

Viskosität (mPa.s): 517

Epoxid-Gehalt (val/kg): 5,20

Farbzahl (Hazen): 60.

## Ansprüche

1. Verfahren zur Herstellung von cycloaliphatischen Diepoxiden der folgenden Formel

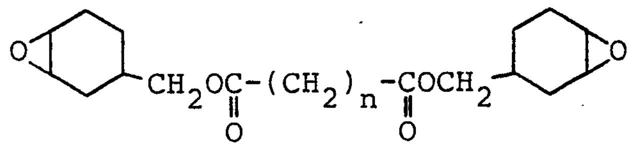

durch Epoxydierung von Diolefinen der Formel

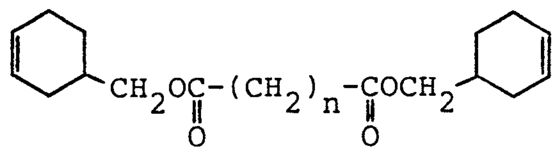

in der n eine Zahl zwischen 0 und 30 ist, mit einer Percarbonsäure in organischer Lösung, dadurch gekennzeichnet, daß man Diolefine der obengenannten Formel mit einer Lösung von Perpropionsäure in Benzol im Molverhältnis 1 : 2 bis 1 : 3,0 (Diolefin zu Perpropionsäure) bei einer Temperatur von 10 bis 100 °C umsetzt.

2. Verfahren nach Anspruch 1, dadurch gekennzeichnet, daß man Diolefine der obengenannten Formel, in der n die Werte 0 bis 1 , vorzugsweise 4, besitzt, einsetzt.

3. Verfahren nach den Ansprüchen 1 und 2, dadurch gekennzeichnet, daß die Perpropionsäurelösung maximal einen Gehalt von 1,5 Gewichtsprozent Wasserstoffperoxid, 1,5 Gewichtsprozent Wasser und ca. 800 ppm Mineralsäure besitzt.

4. Kontinuierliches Verfahren nach den Ansprüchen 1 bis 3, dadurch gekennzeichnet, daß man das cycloaliphatische Diolefin mit einer Lösung von Perpropionsäure in Benzol im Molverhältnis 1 : 2 bis 1 : 3 in ein Reaktionssystem einspeist, das aus einer Folge von 1 bis 4 ideal durchmischten Reaktoren und einem Nachreaktor besteht, die Reaktion bei einer Temperatur von 10 bis 100 °C durchführt, wobei man die Verweilzeit so einstellt, daß der Umsatz, bezogen auf eingesetzte olefinische Doppelbindungen, nach dem oder den ideal durchmischten Reaktoren bei mindestens 80 Molprozent und nach dem Nachreaktor bei mindestens 95, bevorzugt 98 Molprozent, liegt, und daß man das aus dem Nachreaktor austretende Gemisch in einer Kombination von Destillations-und Desorptionsschritten von Benzol, Propionsäure, geringen Mengen an Perpropionsäure and von anderen Leichtsiedern befreit.

5. Verfahren nach den Ansprüchen 1 bis 4, dadurch gekennzeichnet, daß man die Destillations- und Desorptionsschritte unter vermindertem Druck von 0,5 bis 600 mbar bei Temperaturen des Heizmediums von 50 bis 150 °C und bei Verweilzeiten von maximal 10 Minuten, bevorzugt maximal 5 Minuten, in den einzelnen Schritten durchführt.

6. Verfahren nach den Ansprüchen 1 bis 5, dadurch gekennzeichnet, daß man die Destillations- und Desorptionsschritte bei 10 bis 300 mbar durchführt.

7. Verfahren nach den Ansprüchen 1 bis 6, dadurch gekennzeichnet , daß man zunächst Benzol und Propionsäure sowie die geringen Mengen Perpropionsäure größtenteils destillativ abtrennt, worauf man die verbliebene Menge Propionsäure im Rohepoxid mit Benzoldampf weiter desorptiv entfernt und entweder hieran direkt anschließend das Benzol und Spuren von Propionsäure desorptiv mit Wasserdampf und/oder Inertgasen austreibt, oder daß man nach der Desorption mit Benzoldampf das rohe Epoxid zunächst mit wäßrigen Alkalien und anschließend mit Wasser wäscht und erst dann die Desorption mit Wasserdampf und/oder Inertgasen anschließt.

8. Verfahren nach den Ansprüchen 1 bis 7, dadurch gekennzeichnet, daß man das aus der Kombination von Destillations-und Desorptionsschritten erhaltene Gemisch aus Benzol, Propionsäure, geringen Mengen Perpropionsäure, sowie gegebenenfalls anderen Leichtsiedern in eine aus zwei oder mehreren Destillationskolonnen bestehende Destillationsanlage führt und in dem ersten Destillationsschritt über Kopf Benzol, gegebenenfalls im Gemisch mit anderen Leichtsiedern, abzieht, das man gegebenenfalls nach destillativer Reinigung in das Herstellungsverfahren der Perpropionsäure wieder zurückführt, und daß man im Sumpf die Gesamtmenge an Perpropionsäure und Propionsäure, sowie Anteile von Benzol in Mengen von 5 bis 35 Gewichtsprozent, bezogen auf die Sumpfmischung, entnimmt und diese Mischung in

eine zweite Destillationsstufe führt, in der man die Gesamtmenge des darin enthaltenen Benzols und der Perpropionsäure mit Anteilen an Propionsäure über Kopf abzieht und dabei eine Konzentration von Perpropionsäure in dem Kopfprodukt von mehr als 25 Gewichtsprozent nicht überschreitet, dieses Kopfprodukt in das Herstellungsverfahren der Perpropionsäure oder in die Umsetzung der Perpropionsäure mit Olefin zurückführt, und daß man die Perpropionsäure als Sumpfprodukt, gegebenenfalls dampfförmig, oberhalb des Sumpfes abzieht und in das Herstellungsverfahren der Perpropionsäure zurückführt.

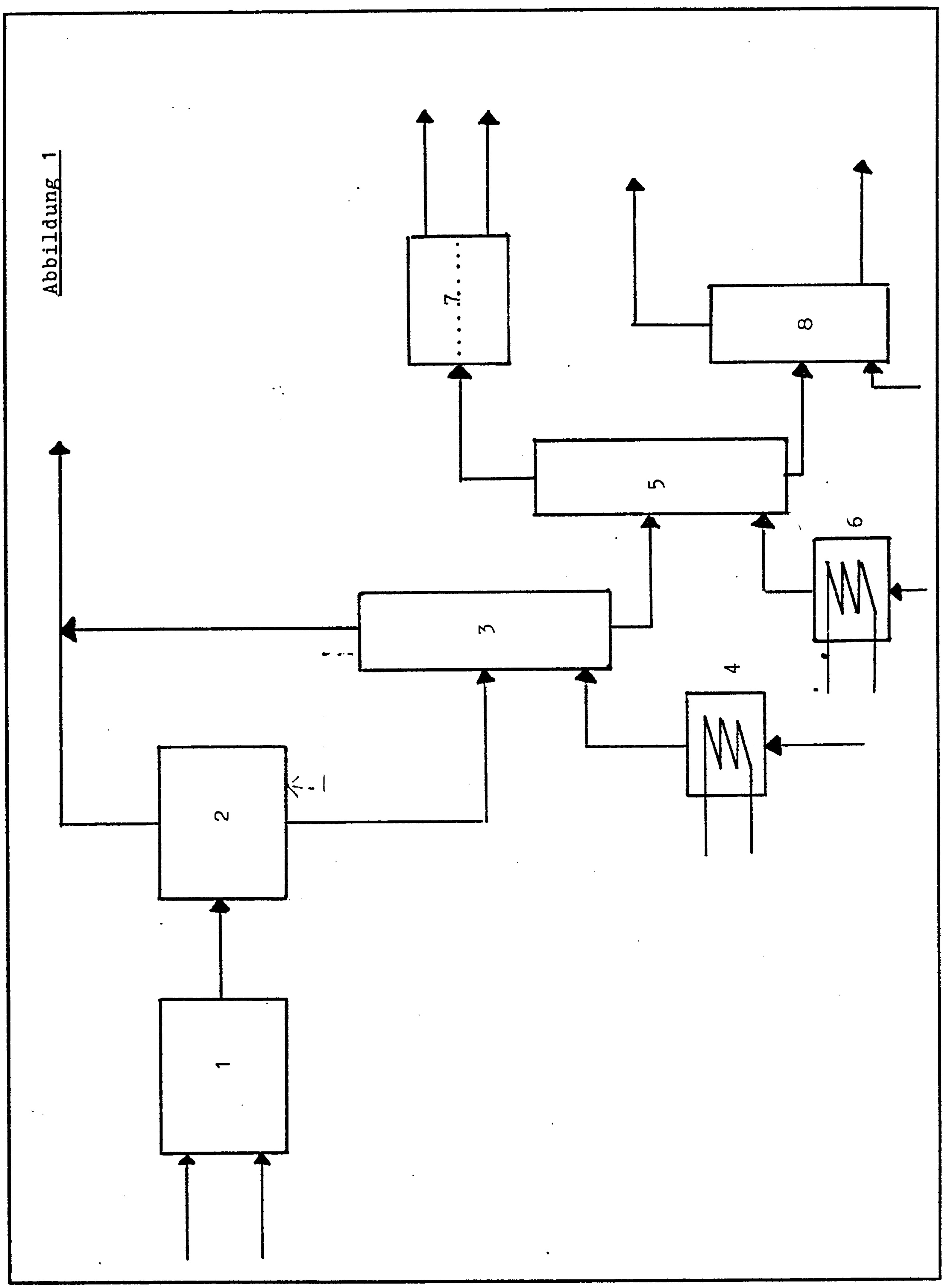
Abbildung 1
1
2
3
4
5
6
7
8

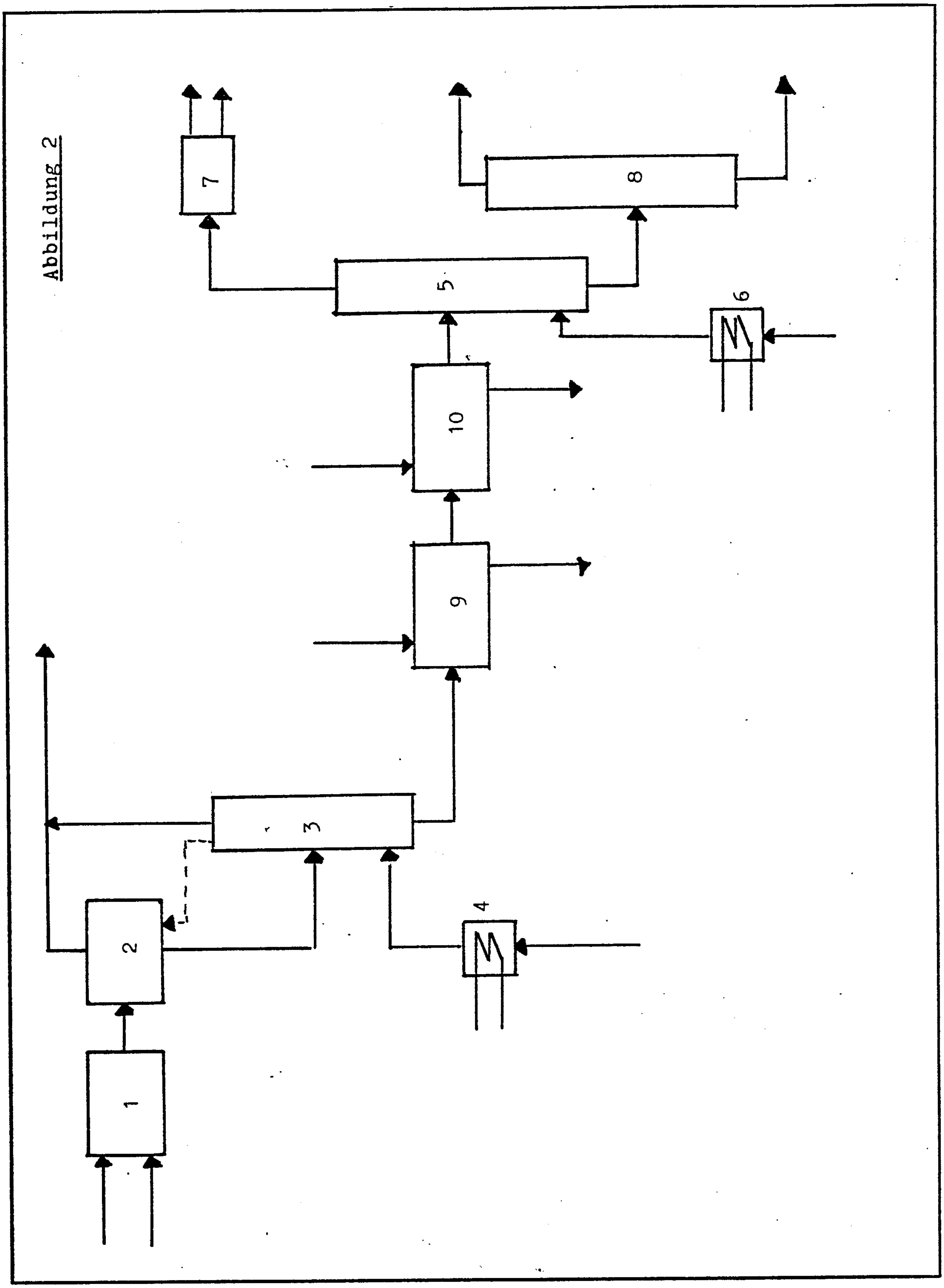


Abbildung 2

01-4-1530 1179

Abbildung 3

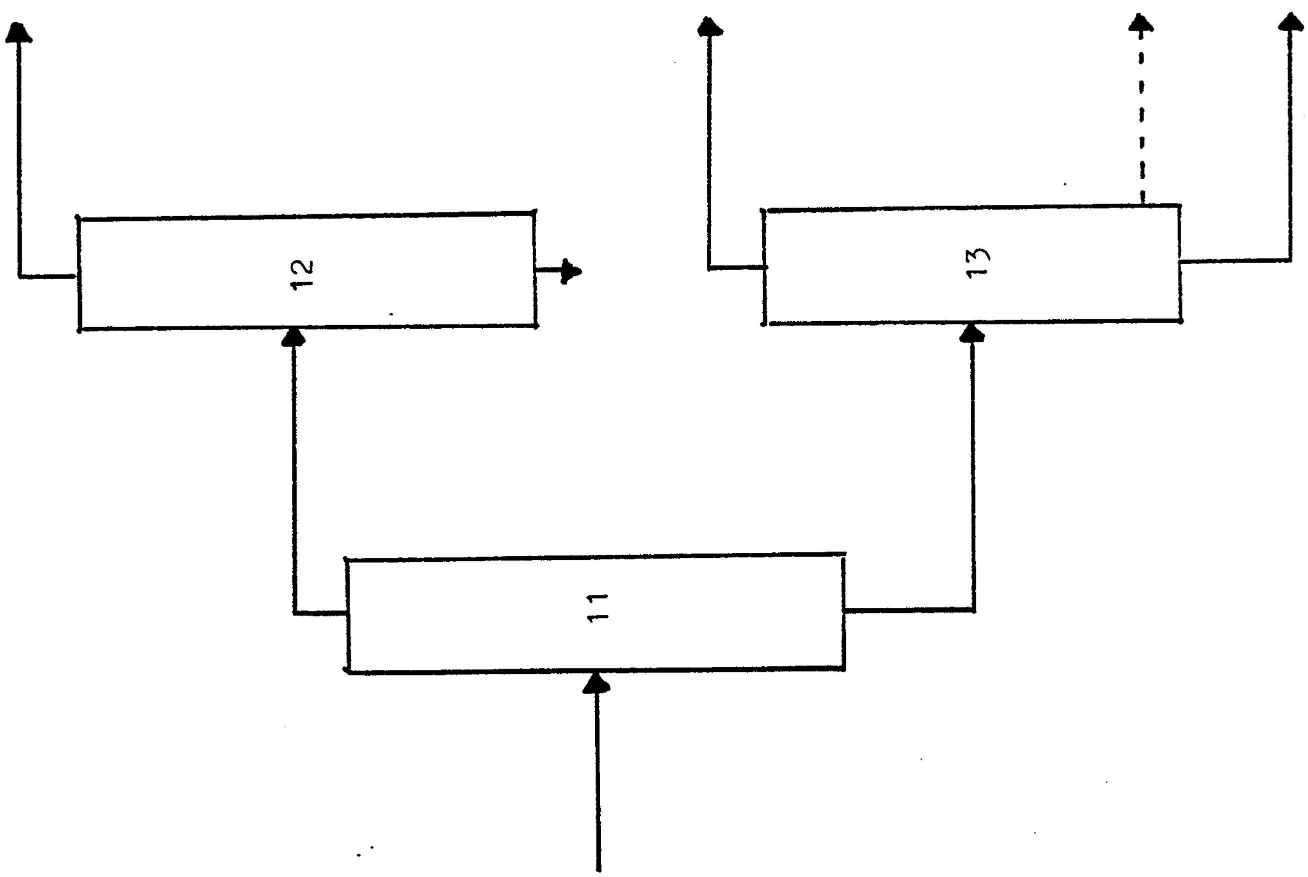


01-4-1530 1179